# EUROPEAN PATENT SPECIFICATION

(11) **EP 3 915 635 B1**
(45) Date of publication and mention of the grant of the patent: **18.11.2026**
(21) Application number: 20744759.0
(22) Date of filing: 23.01.2020
(51) Int. Cl.: A61N 5/06, A61L 2/10

(54) **LIGHT IRRADIATION DEVICE**
LICHTBESTRAHLUNGSVORRICHTUNG
DISPOSITIF D'IRRADIATION DE LUMIÈRE

(30) Priority: 23.01.2019 US 201962795730 P; 29.03.2019 US 201962825993 P; 21.01.2020 US 202016747688
(43) Date of publication of application: 01.12.2021
(73) Proprietor: Seoul Viosys Co., Ltd., Ansan-si, Gyeonggi-do 15429 (KR)
(72) Inventor: BAE, Hee Ho, Ansan-si Gyeonggi-do 15429 (KR); YOON, Yeong Min, Ansan-si Gyeonggi-do 15429 (KR); LEE, A Young, Ansan-si Gyeonggi-do 15429 (KR)
(74) Representative: Stolmár & Partner Patentanwälte PartG mbB
(86) International application number: PCT/KR2020/001167
(87) International publication number: WO 2020/153788

(56) References cited:
- WO-A1-2009/004412
- WO-A2-2006/081312
- KR-A- 20180 067 499
- KR-B1- 101 515 992
- US-A1- 2003 153 962
- US-A1- 2016 015 962
- US-A1- 2018 093 107
- US-A1- 2018 093 107

## Description

### [Technical Field]

Embodiments of the present disclosure described herein relate to a light irradiation device, and more particularly, relate to a light irradiation device used for medical treatment.

### [Background Art]

Recently, various treatment devices using ultraviolet (UV) light have been developed. In general, it is well known that the UV light has a sterilization effect. A conventional UV treatment device employs a traditional UV lamp, operates the UV lamp near a skin, and irradiates the UV light to a part of the skin to be treated.

For example, US 2018/093107 Al discloses a device for disinfecting wounds and burns with a mixture of blue and UV light.

However, the UV light causes an adverse effect such as skin aging or cancer, in addition to the sterilization effect. Accordingly, there is a need to provide a process for obtaining the sterilization effect safely without any influence exerted on a human body.

### [Disclosure]

### [Technical Problem]

Embodiments of the present disclosure provide a light irradiation device capable of obtaining a higher sterilization effect while minimizing an adverse effect on a human body.

### [Technical Solution]

A light irradiation device includes a housing, a substrate provided in the housing, and a light source mounted on the substrate. The light source includes a first light source including at least one first light source and configured to emit first light having a blue wavelength band, a second light source including at least one second light source and configured to emit second light having a ultraviolet wavelength band, and a control unit to control light emission of the first light source and the second light source such that the first light and the second light are emitted, sequentially, or at times close to each other, even if the first light is superposed with the second light or is not superposed with the second light. A dose of the second light source is less than 1/10 of a dose of the first light source.

The control unit controls the first light source and the second light source to emit the second light after starting to emit the first light.

According to an exemplary embodiment, the second light may correspond to at least one of UVA (ultraviolet A), UVB (ultraviolet B), and UVC (Ultraviolet C) wavelength bands.

According to an exemplary embodiment, the first light may have a wavelength band in a range of about 400 nm to about 500 nm. The first light may further include light having a wavelength band corresponding to a visible light, and the first light may have a wavelength band in a range of about 380 nm to about 780 nm.

According to an exemplary embodiment, the second light may have a wavelength band in a range of about 240 nm to about 280 nm.

According to an exemplary embodiment, the first light may be irradiated for a first time, and the second light may be irradiated for a second time shorter than the first time. According to an exemplary embodiment, the second light may be started to be irradiated after the first light is completely irradiated, the second light may be started to be irradiated before the first light is completely irradiated, and at least a portion of the first time and the second time may have a mutually overlapping duration. In addition, the first light may be continuously irradiated, and the second light may be discontinuously irradiated. According to an embodiment of the present invention, the second light may be periodically irradiated.

According to an exemplary embodiment, the light irradiation device may be used to treat a human body. For example, the light irradiation device may be used to treat acute wound.

According to an exemplary embodiment, on the assumption that a dose, which is in a harmless range, per day is an allowable dose when the second light is applied to a human body, the second light source may emit the second light within the allowable dose. According to an exemplary embodiment, the second light may be irradiated with a dose in a range of about 30 J/m2 to about 1,000,000 J/m2.

### [Advantageous Effects]

As described above, according to embodiments of the present disclosure, there is provided a light irradiation device capable of obtaining a higher sterilization effect while minimizing adverse effect on a human body.

### [Description of Drawings]

Figure 1 is a plan view illustrating a light irradiation device, according to an embodiment of the present disclosure;
Figure 2 is a block diagram illustrating a light irradiation device, according to an embodiment of the present disclosure;
Figures 3a and 3b illustrate a non-claimed method for driving a light irradiation device, according to an embodiment of the present disclosure, and illustrate times based on turning on/off the first and second light sources;
Figures 4a and 4b are views illustrating a non-claimed method for driving a light irradiation device, according to an embodiment of the present disclosure, when first light and second light are sequentially irradiated, and illustrates times based on turning on/off the first and second light sources;
Figures 5a to 5c illustrate a non-claimed method for driving a light irradiation device, according to an embodiment of the present disclosure, and illustrates times based on turning on/off the first and second light sources;
Figure 6 is a spectrum of a light emitted from a first light source in a light emitting device according to an embodiment of the present disclosure;
Figure 7a is a plan view of the light irradiation device 100 according to an embodiment of the present disclosure; and Figure 7b is a sectional view taken along line I-I' of Figure 7a;
Figures 8 and 9 illustrate an example in which a light device according to an embodiment of the present disclosure is implemented as a product;
Figure 10 is a graph illustrating a sterilization effect depending on irradiation conditions when a light is irradiated to a sterilization subject using a conventional light emitting device and a light emitting device according to an embodiment of the present disclosure;
Figure 11a is a graph illustrating a test result of sterilization power of first light and Figure 11b is a graph illustrating a test result of sterilization power of second light;
Figure 12a illustrates a bacteria count when first light is individually irradiated, when second light is individually irradiated, and when both the first light and the second light combined are irradiated and Figure 12b illustrates sterilization power when first light is individually irradiated, when second light is individually irradiated, and when both the first light and the second light combined are irradiated;
Figure 13a illustrates a bacteria count irradiated with light obtained by differently setting the sequence of combining first light and second light and Figure 13b illustrates sterilization power obtained by differently setting the sequence of combining the first light and the second light;
Figure 14a illustrates a bacteria count as a function of an amount of the first light under in vitro condition when first light and second light were sequentially irradiated and Figure 14b illustrates sterilization power as a function of the amount of the first light under in vitro condition when the first light and the second light were sequentially;
Figure 15a illustrates a bacteria count as a function of an amount of the first light under in vivo condition, when first light and second light were sequentially irradiated and Figure 15b illustrates the sterilization power as a function of an amount of the first light under in vivo condition, when the first light and the second light were sequentially irradiated;
Figure 16 is a graph illustrating the variation in sterilization power based on days under in vivo condition;
Figure 17 is a graph illustrating the measurement result of the number of the bacteria based on days under in vivo condition;
Figure 18 is a graph illustrating the variation in an area of a wound based on days under in vivo condition;
Figures 19a and 19b are photographs obtained by capturing images of the shape of the wound area based on days, in which Figure 19a is a photograph of wounds in a non-irradiation group, and Figure 19b is a photograph of wounds in the light irradiation group;
Figure 20a is a graph illustrating the percentage of a thymine dimer in a tissue; and Figure 20b illustrates a light emission degree of a part stained with DCFH-DA.

### [Best Mode]

The invention is defined in the claims; other embodiments, methods, items, etc. not being a part of the invention.

Hereinafter, exemplary embodiments of the present disclosure will be described in more detail with reference to accompanying drawings.

The present disclosure relates to a light irradiation device capable of sterilizing a target by applying a sterilizing light to the target. In particular, according to an embodiment of the present disclosure, the light irradiation device may be used to treat a wound. When a target to be sterilized is a human body and the skin of the human body is wounded, it is necessary to sterilize a pathogen at the wounded part. A sterilizing device according to one embodiment of the present disclosure may be used to sterilize the pathogen in the wound. In this case, the pathogens refer to microorganisms such as bacteria, viruses, germs, fungi, protists, or moulds. According to embodiments of the present disclosure, the light irradiation device may be used for various wounds such as a wound, an ulcer, surgical site infection, a laceration, an incised wound, or a punctured wound.

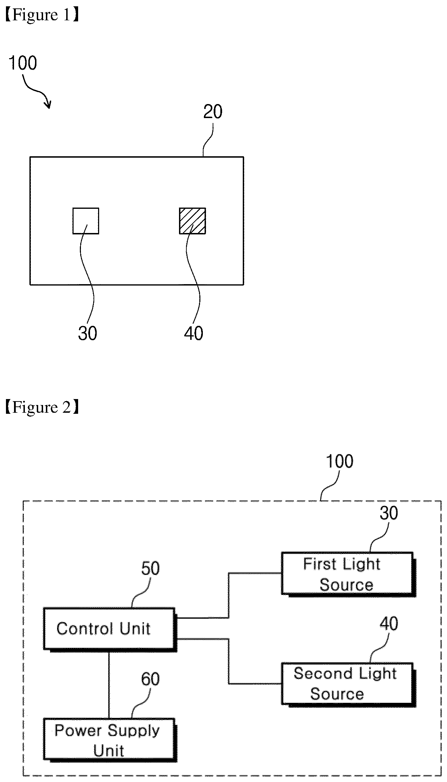

Figure 1 is a plan view illustrating a light irradiation device, according to an embodiment of the present disclosure.

According to embodiments of the present disclosure, a light irradiation device 100 includes a first light source 30 to emit first light, a second light source 40 to emit second light, and a substrate 20 to mount the first and second light sources 30 and 40 thereon.

Since the first light source 30 and the second light source 40 are mounted on the substrate 20, the substrate 20 is not limited to a specific form and may be provided in various forms sufficient to mount the first and second light sources 30 and 40 thereon. The substrate 20 may be provided in the form of including a wiring to supply power to the first and second light sources 30 and 40. In some embodiments, the substrate 20 may include, for example, a metallic substrate or a printed circuit board including the wiring.

The first light source 30 may emit first light in a blue wavelength band of a visible light wavelength band. The first light may correspond to light in a wavelength band of about 400 nm to about 500 nm. In some embodiments of the present disclosure, the first light may have a wavelength band of about 400 nm to about 420 nm. More specifically, the first light may have a wavelength of 405 nm.

The first light acts on a photosensitizer present in microorganisms such as bacteria, germs, and moulds to damage the cell, thereby result in the death of the microorganisms. The first light corresponds to the absorption wavelength of porphyrin, which is a photosensitizer present in bacteria. The first light exhibits higher sterilization power, particularly, in the wavelength range of 400 nm to 420 nm. Additionally, the first light may be in the wavelength range of 455 nm to 470 nm, which corresponds to the absorption wavelength band of the porphyrin, which is the photosensitizer. The porphyrin is a pigment that is essential for the process of intracellular oxygen transfer. The porphyrin exhibits a higher absorption, particularly, in the wavelength range of about 402 nm to about 420 nm, and more particularly absorbs a wavelength in the range of about 455 nm to 470 nm. In an embodiment of the present disclosure, since the content of the porphyrin varies depending on the type of bacteria, the porphyrin may be used for destroying specific bacteria by adjusting the wavelength and the intensity of the first light. When the first light is applied to bacteria, the porphyrin in the bacteria absorbs the first light, and reactive oxygen species are produced in the cell of the bacteria due to the energy of the first light. The reactive oxygen species are accumulated in cells of the bacteria to oxidize cell walls of the bacteria, thereby destroying the bacteria.

The second light source 40 emits the second light in the UV wavelength band. In other words, the second light may be light having a wavelength band in the range of about 100 nm to about 400 nm, and may be UVA, UVB, or UVC. The UVA may have a wavelength band in the range of about 315 nm to about 400 nm, the UVB may have a wavelength band in the range of about 280 nm to about 315 nm, and the UVC may have a wavelength band in the range of about 100 nm to about 280 nm. In some embodiments of the present disclosure, the second light may correspond to the UVC, and may have a wavelength band in the range of about 240 nm to about 280 nm. More specifically, the second light may have the wavelength of 275 nm.

When the second light is applied to bacteria, the DNA in the bacteria absorbs the second light, and the DNA structure may be changed due to the energy of the second light. The absorption of light by the DNA causes the binding of thymine and adenine in the DNA to be broken. This is because a base such as purine or pyrimidine, which constitutes the DNA, strongly absorbs UV light. As a result of light absorption, a thymine dimer is formed. This process leads to the DNA mutation, and the mutated DNA causes the death of the bacteria since the mutated DNA has no ability of cell proliferation. The DNA may absorb light having a wavelength band in the range of about 240 nm to about 280 nm.

Figure 2 is a block diagram illustrating the light irradiation device, according to an embodiment of the present disclosure.

Referring to Figure 2, according to embodiments of the present disclosure, the light irradiation device 100 may include the first light source 30 to emit first light, the second light source 40 to emit second light, a control unit 50 to control the first light source 30 and the second light source 40, and a power supply unit 60 to supply power to the control unit 50 and the first and second light sources 30 and 40.

Each of the first and second light sources 30 and 40 may emit the first light including a blue wavelength band and the second light including a UV wavelength band, as described above. The first and second light sources 30 and 40 may be implemented with various types of light sources. For example, each of the first and second light sources 30 and 40 may independently use various types of light sources such as a light emitting diode, a halogen lamp, a fluorescent lamp, a gas discharge lamp, or a laser, and the types of the light sources are not limited.

The control unit 50 may control whether light is emitted from the first and second light sources 30 and 40, an amount of the light, the intensity of the light, or a time in which the light is emitted.The control unit 50 may control whether the light is emitted, an amount of the light, the intensity of the light, or the time in which the light is emitted, through various manners.

The power supply unit 60 is electrically connected with the first and second light sources 30 and 40 and the control unit 50 to supply power to the first and second light sources 30 and 40 and the control unit 50. Although the drawings illustrate that the power supply unit 60 supplies power to the first and second light sources 30 and 40 via the control unit 50, the present disclosure is not limited thereto. In other embodiments, the power supply unit 60 may be directly connected with the first and second light sources 30 and 40 to supply power to the first and second light sources 30 and 40.

The light irradiation device 100 may further include an optical unit (not shown) to selectively collect or radiate light emitted from the first and second light sources 30 and 40. The optical unit may focus the light generated from the first and second light sources 30 and 40 to a small area, or a large area as necessary. Alternatively, the light may be focused or dispersed in a uniform or non-uniform depending on a position where the light is irradiated. The optical unit may include at least one lens, and the lens may perform various functions of focusing, dispersing, homogenizing, or nonhomogenizing light from the first and second light sources 30 and 40.

For example, when a light is irradiated in the small area using the light emitting device 100, a lens for focusing the light from the first and second light sources 30 and 40 may be used. On the contrary, when a light is provided in the large area, for example, an entire room using the light emitting device 100 according to an exemplary embodiment, a lens for dispersing the light may be used.

In the present embodiment, the control unit 50 simultaneously, or individually drives the first light source 30 and the second light source 40. In other words, the first and second light sources 30 and 40 may be turned on/off simultaneously, or individually. In addition, even the intensities of light, that is, the first light and the second light emitted from the first and second light sources 30 and 40 may be simultaneously, or individually controlled.

In an embodiment of the present disclosure, the control unit 50 may allow a daily irradiation amount to be 3 mJ/cm² or less. In particular, in the case of UVC, the control unit 50 maintains the daily irradiation amount to be 3 mJ/cm² or less. Further, in the case of UVA, when a daily irradiation time is less than 1,000 seconds, the daily irradiation amount is maintained such that the daily irradiation amount does not exceed 1 J/cm², and when the daily irradiation time is equal to or greater than 1,000 seconds, the daily irradiation amount is maintained such that the daily irradiation amount does not exceed 1 mW/cm².

In an embodiment of the present disclosure, the distance from the first and second light sources 30 and 40 to a target to be sterilized may be variously set. For example, the distance may vary depending on the intensities of light from the first and second light sources 30 and 40, the type of the target to be sterilized, an area or a volume to be sterilized, or a target material (for example, germs or bacteria) to be sterilized. Similarly, in an embodiment of the present disclosure, the time in which light from the first light source 30 and the second light source 40 is irradiated may be variously set.

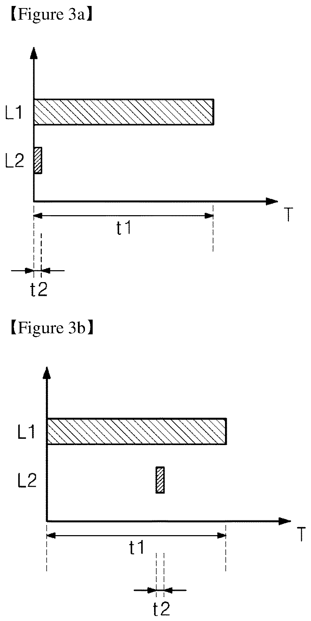

Figures 3a and 3b illustrate a method for driving a light irradiation device, according to some embodiments of the present disclosure, and illustrates various timing based on turning on/off the first and second light sources;

In some embodiments, in the light irradiation device 100, the first light from the first light source 30 is designated as "L1", the second light from the second light source 40 is designated as "L2", and an elapsed time is designated as "T". The first light source 30 is turned on for a first time t1 to emit the first light L1, and the second light source 40 is turned on for a second time t2 to emit the second light L2. In some embodiments, the first time t1 in which the first light L1 is irradiated may be longer than the second time t2 in which the second light L2 is irradiated. Since the second light L2 exerts a great influence on, especially, a human body, the second light L2 may be irradiated for a shorter time than the irradiation time of the first light L1. For example, the first light L1 may be applied for about 10 minutes, and the second light L2 may be applied for less than about 10 seconds.

The irradiation times t1 and t2 of the first light L1 and the second light L2 emitted from the first and second light sources 30 and 40, and amounts of the first light L1 and the second light L1 in irradiation may vary, but a total dose applied to the target to be sterilized may be set to a value harmless to the human body. In particular, a predetermined dose per day, which is in a harmless range, may be an allowable dose when the second light L2 is applied to the human body, and then the second light source 40 may emit the second light L2 less than the allowable dose. The dose may vary depending on the harmfulness of the light emitted from the first light source 30 and the second light source 40. In some embodiments, the dose of the second light source 40 may be less than 1/10 of the dose of the first light source 30, and, according to another embodiment may be 1/20 of the dose of the first light source 30. For example, the allowable dose of the second light L2 may be in the range of about 30 J/m² to about 1,000,000 J/m².

As illustrated in Figure. 3a the first light L1 and the second light L2 start to be irradiated at mutually different times, wherein the first light L1 is first irradiated.

The times in which the first light L1 and the second light L2 are irradiated may overlap, or may not overlap. When the times, in which the first light L1 and the second light L2 are irradiated, do not overlap, the interval between the times in which the first light L1 and the second light L2 are applied may be set to be a shorter time interval. For example, the interval between the times in which the first light L1 and the second light L2 are applied may be within several hours, several minutes, or several seconds.

The sterilizing device according to some embodiments of the present disclosure exhibits a sterilization effect higher than the individual sterilization effect by the first light L1 or the individual sterilization effect by the second light L2, due to the synergy effect that may be obtained as the first light and the second light are applied simultaneously, or within the times close to each other.

In some embodiments, the sterilizing device employs the sterilization principle of the first light of generating reactive oxygen species due to a photosensitizer and the second light of causing the damage to DNA by obtaining a thymine dimer. In some embodiments, the significantly high sterilization effect may be obtained within a shorter time even with a smaller amount of energy by using the first light source and the second light source, as compared to the case of an individual use of the first and second light sources.

The death rate of the bacteria having received chemical and physical stresses may rapidly increase even due to a weak stimulus additionally applied thereto. Accordingly, in some embodiments, mutually different two sterilizing mechanisms based on the first light and the second light, which correspond to blue light and UV light, apply mutually different stresses to the bacteria. Accordingly, the synergy effect of the stresses may destroy the bacteria with a smaller amount of energy as compared to the individual use of the two light sources. In some embodiments, the second light is irradiated in the amount harmless to a biological tissue of the target, which is to be sterilized, while being applied together with the first light. Accordingly, the sterilization synergy effect may be obtained by two light sources, so the present disclosure may produce the effective sterilization effect within a shorter time without the damage to a human tissue, when the target to be sterilized is a human body.

To the contrary, the use of only the first light is not harmful to the human body, but the sterilization power may be weak. Accordingly, the first light needs to be irradiated with higher energy for a longer time. The use of only the second light produces excellent sterilization power, but it may be harmful to the human body.

As described above, in some embodiments of the present disclosure, the sterilizing device may be used to sterilize various pathogens. Particularly, the light irradiation device 100 may be used for sterilizing infectious bacteria in the initial stage by irradiating sterilizing light to an acute infected wound, and thus, the period for curing the wound may be short. For the acute wound, reducing an infectious bacteria count in the initial stage of the wound is the most important in the curing process of the acute wound. When the initial sterilization is not sufficiently performed with respect to the acute wound, the curing of the cut may not be fully perform. A cut may develop into a chronic cut that may not cured for 3 months or longer. However, when the infectious bacteria are sterilized in the initial stage using the light irradiation device 100, the chronic cut may be prevented.

In addition, microorganisms, such as bacteria, germs, and moulds, present on animals and various articles may be sterilized in addition to the human body. Accordingly, the target to be sterilized by the sterilizing device according to an embodiment of the present disclosure is not limited to a human body, but it may apply to animals and various articles.

According to some embodiments of the present disclosure, as described above, the sterilization effect may significantly increase when both the first light and the second light emitted from the first light source 30 and the second light source 40 are applied simultaneously, or within the times close to each other. In addition, in some embodiments, when the first light and the second light are sequentially irradiated in that order, the significantly higher sterilization effect may be obtained as compared to the order that the second light and the first light are sequentially irradiated. Accordingly, the sterilization effect may be maximized through sequentially applying the first light, and then the second light to the target to be sterilized.

According to some embodiments of the present disclosure, the first light is applied to the target to be sterilized for a specific time before the second light is irradiated, and then the second light is irradiated. Accordingly, DNA is prevented from being recovered from the damage again after the first light is first irradiated. Accordingly, the significantly higher sterilization effect may be obtained even with a smaller dose as compared to the case that the first light is individually irradiated. In addition, the second light may have excellent sterilization power for the subject to be sterilized but it may have an adverse effect on the human body, for example, skin aging or cancer, when exposed to the human body for a long time. Therefore, there may be limitations as to applying only the second light to the subject to be sterilized. However, according to embodiments of the present disclosure, the irradiation of the second light in addition to the irradiation of the first light may obtain the significant sterilization effect despite a small amount of the irradiation, in comparison with the irradiation.

In an embodiment of the present disclosure, when the second light is emitted sequentially after the first light, in addition to the first light, an amount of the second light needs to be controlled. In some embodiments, the synergy effect of sterilization may be obtained and an influence on the human body may be minimized by sequentially irradiating the first light and the second light. To this end, when the first light source 30 and the second light source 40 are turned on/off, one or more manner of continuously emitting light, one or more manner of sequentially increasing or decreasing the intensity of light, one or more flickering manner, or a manner of combining the above manners may be employed.

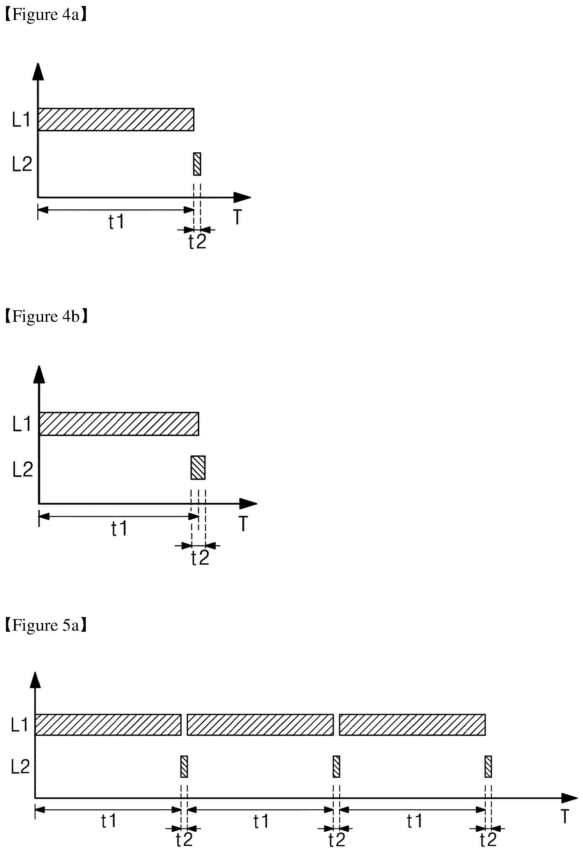

Figures 4a and 4b are views illustrating a method for driving the light irradiation device according to embodiments of the present disclosure, when the first light and the second light are sequentially irradiated, and illustrates different times associated with turning on/off the first and the second light sources. The different times here may include different timings of emitting the first and second light sources, different durations of the irradiation of the first and the second light sources, etc.

Referring to Figures 4a and 4b, the first light L1 is first irradiated, and then the second light L2 is irradiated. When the first light L1 is first irradiated and then the second light L2 is irradiated, the sterilization effect may significantly increase as compared to the case that the second light is first irradiated and then the first light L1 is irradiated. When the second light L2 is first irradiated and the first light L1 is later irradiated, the effect of inhibiting the proliferation of bacteria by the second light L2 may be reduced by irradiating the first light L1. Accordingly, even if the structure of DNA is partially mutated by the second light L2, the mutated DNA may be subject to photoreactivation by irradiating the first light L1. The bacteria recovered through the irradiation of the first light L1 return to a state in which the bacteria may be proliferated. Accordingly, although the total sterilization power is still excellent, the sterilization power in the final stage may be more reduced as compared to the case that the first light L1 and the second light L2 are sequentially irradiated.

On the other hand, when the first light L1 is applied to the target to be sterilized and then the second light L2 is sequentially applied to the target to be sterilized by using the light irradiation device 100, reactive oxygen species are generated in bacteria by the first light L1, which is first irradiated, so oxidative stress is caused in bacteria. In this state, since additional sterilization is performed by the second light L2 irradiated later, the death rate of the bacteria may significantly increase even in a smaller irradiation amount.

In some embodiments, the time point at which the second light L2 is applied may vary in the setting that the first light L1 and the second light L2 are sequentially applied. For example, irradiation of the second light L2 may start after the irradiation of the first light L1 is finished as illustrated in Figure 3a, and as illustrated in Figure 3b, the irradiation of the second light L2 may start even though the irradiation of the first light L1 is not finished. In this case, since time points at which the first light L1 and the second light L2 are irradiated may partially overlap, the first time and the second time may have mutually overlap durations.

As described above, the light irradiation device 100 according to embodiments of the present disclosure may be driven by the control unit 50 in the setting that the first light L1 and the second light L2 are sequentially irradiated.

Figures 5a to 5c illustrate a method for driving a light irradiation device, according to embodiments of the present disclosure, and illustrates different times associated with turning on/off the first and second light sources;

Referring to Figure 5a, the first light L1 and the second light L2 may be periodically irradiated to the target to be sterilized. In other words, the first light L1 is irradiated to the target to be sterilized for the first time t1, and the second light L2 is irradiated to the target to be sterilized for the second time t2. Then, irradiation of the first light L1 and the second light L2 is repeated. The pattern of the repeat period and the repeat count may vary depending on the type of the target to be sterilized and the total amount of the target to be sterilized. In this case, the repeat period and the repeat count of the first light L1 and the second light L2 may be determined such that the total dose of the first light L1 and the total dose of the second light L2 become values equal to or less than the allowable dose for the human body in order to avoid harmful effects.

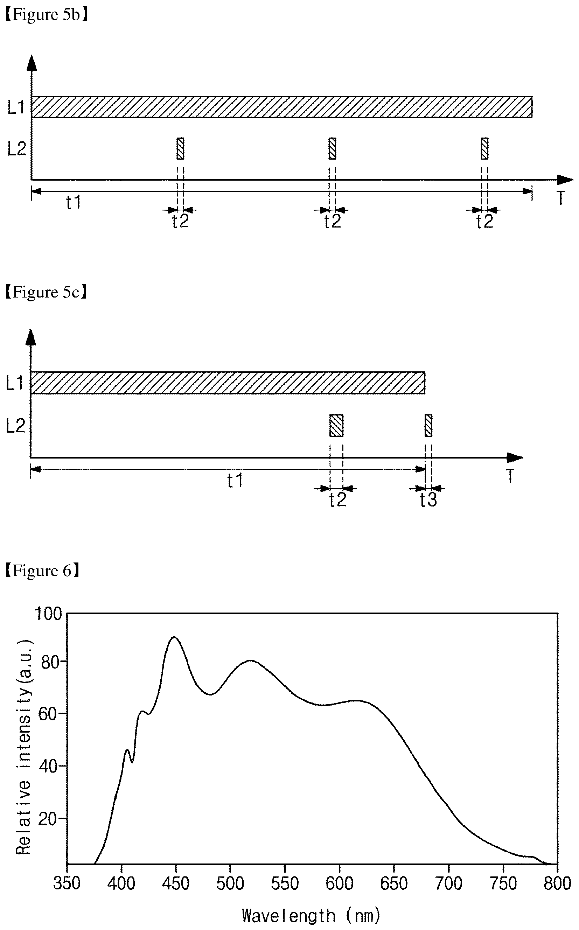

Referring to Figure 5b, when the first light L1 and the second light L2 are applied, the first light L1 may be continuously applied to the target to be sterilized without interruption under the condition that the second light L2 is applied after the first light L1 is applied. To the contrary, the second light L2 is not continuously applied, but discontinuously applied while being superposed with the first light L1.

As illustrated in drawings, the first light L1 may be continuously applied to the target to be sterilized for the first time t1 without interruption, and the second light L2 may be applied to the target to be sterilized for the second time t2 during the continuous application of the first light L1, after the first light L1 is applied to some extent. The second light L2 may be continuously repeatedly applied to the target to be sterilized.

Referring to Figure 5c, when the first light L1 and the second light L2 are applied, the first light L1 may be continuously applied to the target to be sterilized without interruption or may be stopped before the second light L2 is applied, under the condition that the second light L2 is applied after the first light L1 is applied. As illustrated in drawings, when the first light L1 is applied to the target to be sterilized for the first time t1, the second light L2 may be applied for the second time t2 during the application of the first light L1. Thereafter, after the application of the first light L1 is finished, the second light L2 may be applied for a third time t3. In this case, regarding the application time of the second light L2, the second light L2 may be applied to the target to be sterilized for mutually different times within an allowable dose permitted as being safe for a human body. In other words, the second time t2 and the third time t3 in which the second light L2 is applied may have mutually different values, as shown.

In some embodiments of the present disclosure, when the second light L2 is applied as soon as the first light L1 is applied and stopped, the highest sterilization effect may be exhibited, and the second light L2 may be sequentially applied without interruption in the state the first light L1 is applied. However, instead of that the second light L2 is applied as soon as the first light L1 is applied and stopped, time may be slightly elapsed after the first light L1 is applied and stopped and then the second light L2 may be applied. In this case, the elapsed time interval may be significantly short. Meanwhile, when the sterilization effect is obtained as the first light L1 and the second light L2 are sequentially applied, the next sequential irradiation of the first light L1 and the second light L2 may be performed after a sufficient amount of time is elapsed.

In embodiments of the present disclosure, the first light source includes a blue wavelength sterilizable in the visible light wavelength band, but the first light source is not limited thereto. In other embodiments, the first light source may further include another light of the visible light wavelength band in addition to the blue wavelength band.

Figure 6 is a spectrum of a light emitted from a first light source in a light emitting device according to embodiments of the present disclosure.

Referring to Figure 6, the first light source emits the light in a wavelength band of about 380 nm to about 750 nm, most of which corresponds to a visible light wavelength band. That is, the first light source corresponds to a light source which emits white light. In sine embodiments, the first light source includes a light in the blue wavelength band which is combined with the second light to generate synergy, and thus the above-described sterilization effect may be obtained in the same manner.

In addition, the first light source in the embodiment has a spectrum similar to sunlight having a form in which lights of the entire wavelength bands are evenly mixed. However, the first light source according to embodiments of the present disclosure is different from sunlight because the first light source emits a light except for the most of an ultraviolet wavelength band. The light source according to embodiments of the present disclosure emits a light having a wavelength band of about 380 nm to about 780 nm which substantially correspond to the entire wavelength band of a visible light.

In embodiments of the present disclosure, the phrase, "similar to sunlight" means that an overlapping area based on a normalized solar spectrum is more than a specific value and a deviation of the peak from the normalized solar spectrum (a deviation degree from the peak of the normalized solar spectrum) is lower than a specific value. For example, in an embodiment of the invention, the first light source may emit the light having an area of about 55% or more of an area of the normalized solar spectrum and a peak of the first light may have a deviation of about 0.14 or less from the normalized solar spectrum.

As described above, because the first light may have the spectrum similar to the sunlight, the first light may have an effect similar to an effect of frequent exposure to the sunlight. Therefore, synthesis of vitamin D may be facilitated or prevalence of diseases such as myopia may be lowered.

### [Mode for Invention]

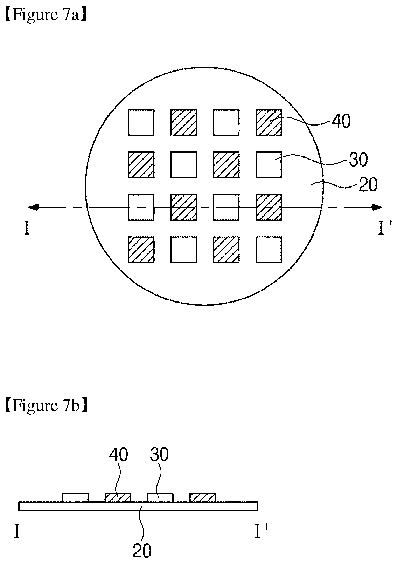

According to embodiments of the present disclosure, the light irradiation device 100 may be implemented in various forms. Figure 7a is a plan view of the light irradiation device 100 according to embodiments of the present disclosure, and Figure 7b is a sectional view taken along line I-I' of Figure 7a.

Referring to Figures 7a and 7b, the light irradiation device 100 may include the first light source 30, the second light source 40, and the substrate 20 on which the first light source 30 and the second light source 40 are mounted.

In some embodiments, a plurality of first light sources 30 may be provided, and a plurality of second light sources 40 may be provided. For example, the first light sources 30 and the second light sources 40 may be provided in equal numbers and may be alternately arranged in the form of a matrix as illustrated in Figure 7a. However, numbers of the first and second light sources 30 and 40 is not limited thereto, and the number of the first light sources 30 may be smaller than the number of the second light sources 40. In addition, according to embodiments of the present disclosure, the first light sources 30 and the second light sources 40 may be regularly or irregularly arranged depending on the number of the first light sources 30 and the number of the second light sources 40.

According to embodiments of the present disclosure, the light irradiation device 100 may further include a housing to receive the first and second light sources 30 and 40 and the substrate 20. The housing may have a transmission window to transmit light emitted from the first and second light sources 30 and 40 and the light emitted from the first and second light sources 30 and 40 may be provided to the human body through the transmission window.

In an embodiment of the present disclosure, the control unit 50 may be provided in various forms on the substrate 20. For example, the control unit 50 may be provided in the form of a separate circuit wiring or in the form of a separate chip, to be mounted on the substrate 20.

As described above, according to an embodiment of the present disclosure, the sterilizing device may be applied to various other devices requiring sterilizing, and particularly, may be applied to a device using a light source. In addition, the sterilizing device may be used as a lighting device in addition to the intrinsic function thereof. For example, according to an embodiment of the present disclosure, the sterilizing device may further include an additional light source for lighting a specific space. In this case, the additional light source may emit light in a visible wavelength band. The additional light source may emit light corresponding to the entire spectrum of the visible light area, or may emit light corresponding to the spectrum of a specific color.

Alternatively, in an embodiment of the present disclosure, the first light source 30 may emit light in the visible light wavelength band including light in the blue wavelength band without an additional light source. For example, the first light source 30 emits light in a wavelength band in the range of about 380 nm to about 750 nm, and most of the light corresponds to a visible light wavelength band. In this case, the first light source 30 may totally provide light in the visible light wavelength band while providing light in the blue wavelength band for obtaining a synergy effect through the combination with the second light source 40, thereby obtaining the sterilization effect as in embodiments described above. In this manner, when an additional light source is provided to emit light in the visible light wavelength band, or the first light source emits the light in the visible light wavelength band, the light may have the spectrum similar to that of sunlight. The light having the spectrum similar to that of sunlight may exhibit the effect similar to being frequently exposed to sunlight. Accordingly, the synthesis of vitamin D may be facilitated or the prevalence ratio of illnesses such as nearsightedness may be lowered.

According to embodiments of the present disclosure, the light irradiation device 100 may be implemented in various forms. Figure 7a is a plan view of the light irradiation device 100 according to embodiments of the present disclosure, and Figure 7b is a sectional view taken along line I-I' of Figure 7a.

Referring to Figures 7a and 7b, the light irradiation device 100 may include the first light source 30, the second light source 40, and the substrate 20 on which the first light source 30 and the second light source 40 are mounted.

In some embodiments, a plurality of first light sources 30 may be provided, and a plurality of second light sources 40 may be provided. For example, the first light sources 30 and the second light sources 40 may be provided in equal numbers and may be alternately arranged in the form of a matrix as illustrated in Figure 7a. However, numbers of the first and second light sources 30 and 40 is not limited thereto, and the number of the first light sources 30 may be smaller than the number of the second light sources 40. In addition, according to embodiments of the present disclosure, the first light sources 30 and the second light sources 40 may be regularly or irregularly arranged depending on the number of the first light sources 30 and the number of the second light sources 40.

According to embodiments of the present disclosure, the light irradiation device 100 may further include a housing to receive the first and second light sources 30 and 40 and the substrate 20. The housing may have a transmission window to transmit light emitted from the first and second light sources 30 and 40 and the light emitted from the first and second light sources 30 and 40 may be provided to the human body through the transmission window.

In an embodiment of the present disclosure, the control unit 50 may be provided in various forms on the substrate 20. For example, the control unit 50 may be provided in the form of a separate circuit wiring or in the form of a separate chip, to be mounted on the substrate 20.

The light emitting device may be implemented in various forms and used for various purposes. For example, the light emitting device according to an embodiment of the inventive concept may be variously applied where lighting and sterilization are required, and in particular, may be used as a lighting device. For example, the light emitting device may be used in the lighting device for medical facilities such as operating rooms and hospitals, for public health, or for personal hygiene. In particular, the lighting device according to an embodiment of the inventive concept may be used for patient treatment.

The lighting device of the inventive concept may applied to public facilities, public use spaces and public use products to be used for public treatment or may be applied to personal facilities, personal use spaces and personal use products to be used for personal treatment.

In addition, the light emitting may not be used exclusively for the lighting device. The light emitting device may be added to anther treatment device to be used.

Hereinafter, a specific embodiment of a lighting device according to an embodiment of the present disclosure will be described.

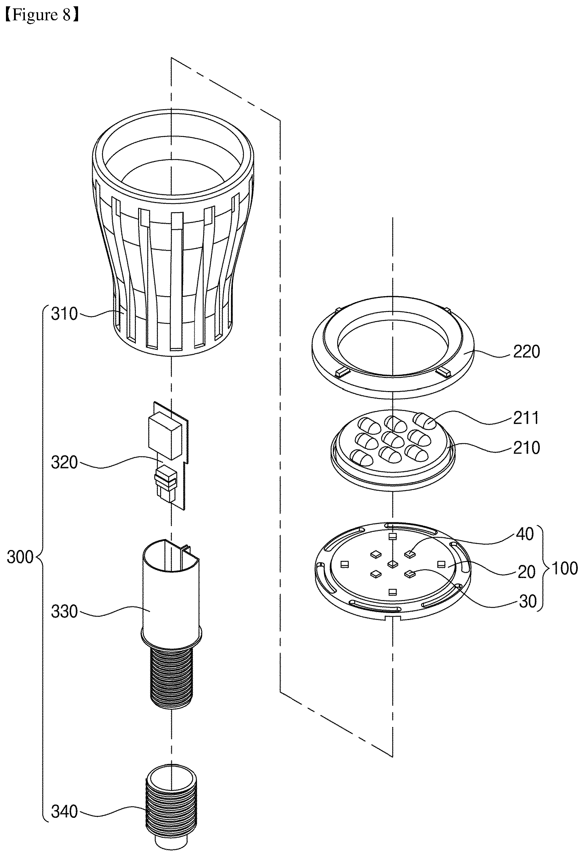

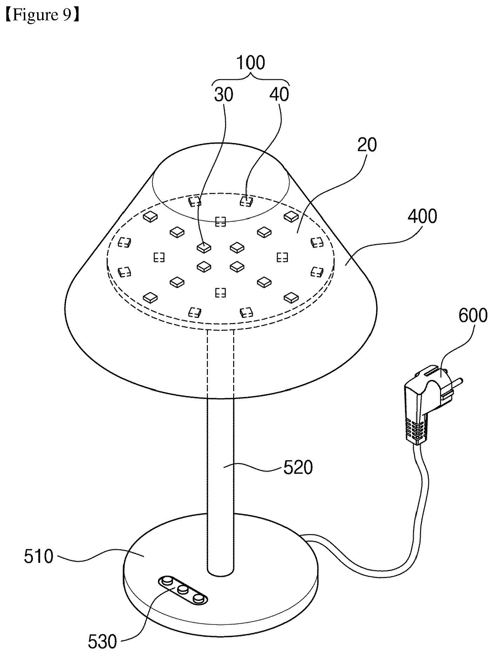

Figures 8 and 9 illustrate an example in which a light device according to an embodiment of the present disclosure is implemented as a product.

Referring to Figure 8, a lighting device according to an embodiment of the present disclosure includes the light emitting device 100 for emitting a light, a housing 300 in which the light emitting device 100 is accommodated, a window 210 provided at an upper part of the light emitting device, and a fixer 220 fixing the window 210 and the housing 300.

The housing 300 accommodates and supports the light emitting device 100 and is not limited as long as the housing 300 supplies electrical power to the light emitting device 100. For example, as shown, the housing 300 may include a main body 310, a power supplier 320, a power case 330, and a power connector 340. The power supplier 320 may be accommodated in the power case 330 and electrically connected to the light emitting device 100 and may include at least one IC chip. The IC chip may adjust, convert or control characteristics of the power supplied to the light emitting device 100.

The power case 330 may accommodate and support the power supplier 320, and the power case 330 in which the power supplier 320 is fixed may be located inside the main body 310.

The power connector 340 may be disposed at a lower end of the power case 330 and may be coupled with the power case 330. Accordingly, the power connector 340 may be electrically connected to the power supplier 320 inside the power case 330 to serve as a path through which external power is supplied to the power supplier 320.

The light emitting device 100 may include the substrate 20 and the first and second light sources 30 and 40 disposed on the substrate 20 and may have a form as the above-described embodiments. The light emitting device 100 may be provided on the upper part of the main body 310 and electrically connected to the power supplier 320. The substrate 20 may have a shape corresponding to the fixer 220 of the upper part of the main body 310 to be stably fixed to the main body 310.

The window 210 may be disposed on the housing 300 to cover the upper part of the light emitting device 100. The window 210 may be disposed on the light emitting device 100 and may be fixed to the main body 310 to cover the light emitting device 100. The window 210 may be provided with a lens member 211 to facilitate diffusion of the light from the light emitting device 100. The window 210 may have a transparent material. A shape and light transmittance of the window 210 may be modified to adjust directivity of the lighting device. Therefore, the window 210 may be modified in various forms depending on the purpose of use and application of the lighting device.

The fixer 220 may be provided on the window 210 to fasten the window 210, the light emitting device 100, and the main body 310 to one another.

The lighting device having the above-described structure may be mounted in various light treatment devices. In addition, the lighting device may be used as a lighting fixture mounted on a wall or a ceiling forming a specific space (e.g., a chamber).

The lighting device according to an embodiment of the present disclosure may be implemented in a form which is used in real life.

Referring to Figure 9, the lighting apparatus 1000' according to another embodiment of the present disclosure may include a pedestal 530, the light emitting device 100 for emitting the light, a supporter 520, and a reflector 400 surrounding the light emitting device 100. The lighting device 1000' according to another embodiment of the present disclosure may be disposed on a variety of treatment devices.

An input unit 530 may be disposed on a surface of the pedestal 510 to control an operation of the lighting device 1000'. The pedestal 510 is connected and fixed through the substrate 20 on which the lighting device 100 is disposed and the supporter 520. The pedestal 510 allows power to be supplied to the light emitting device through a power supplier 600. The supporter 520 may be connected between the pedestal 510 and the substrate 20 on which the light emitting device 100 is disposed and a wire (not shown) for supplying the power may be provided therein.

In the embodiment, the supporter 520 is shown as being formed of a single solid material, but is not limited thereto, and may be made of a bendable material capable of bending at least one time or made of a flexible material to be changed into various shapes. For example, the supporter 520 may have a ductility to be deformed by a specific degree of external force and maintain a shape the supporter 520 when there is no external force. For example, a wiring may receive an external force to be partially changed and the changed wiring 130 may maintain the final shape to which the external force is applied when the external force is removed. To this end, the supporter 520 may be provided in a bellows shape.

The reflector 400 surrounding the light emitting device 100 may be made of a metallic material such as aluminum or the like, which is capable of reflecting a light emitted from the light emitting device and increasing illuminance, or a material capable of transmitting a light. A coating layer including a photocatalyst material may be formed on an inner surface of the reflector 400. The photocatalyst material may include at least one from the group of TiO₂, ZnO, ZrO₂, and WO₃.

Hereinafter, an experimental example of the sterilization effect of the light irradiation device according to an embodiment of the present disclosure will be described.

### Experimental Example 1- Sterilization Effect According to Irradiation Conditions

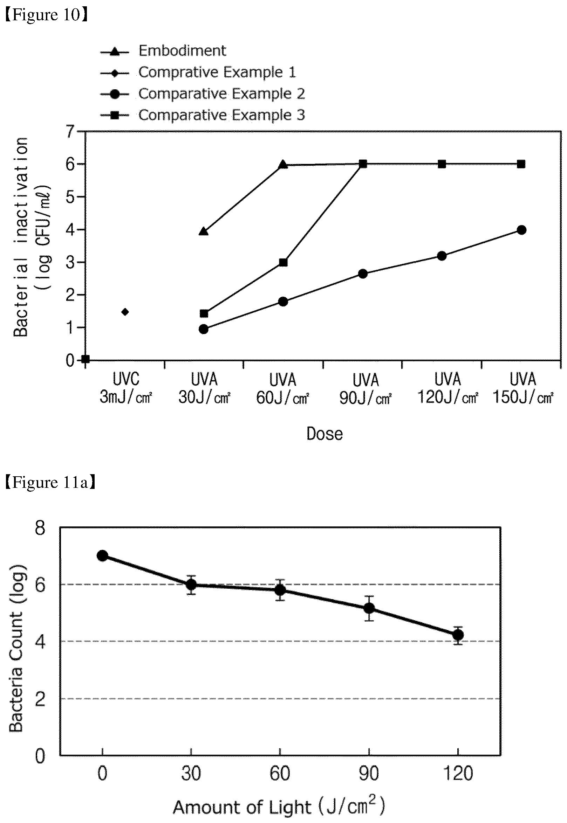

Figure 10 is a graph illustrating a sterilization effect depending on irradiation conditions when a light is irradiated to a sterilization subject using a conventional light emitting device and a light emitting device according to an embodiment of the present disclosure. In Figure 10, a bacterium used as a subject of sterilization is staphylococcus aureus. The staphylococcus aureus was smeared on a bacterial culture medium and incubated at 35 to 37 °C for one day, and bacterial colonies formed on the bacterial culture medium were collected, suspended in a saline solution, and performed with centrifuge, and a supernatant was discarded, a saline solution is added to be diluted, and thus a bacterial solution having a suitable concentration for a sterilization experiment was prepared. The prepared bacterial solution was placed in separate containers, the conventional light emitting device and the light emitting device according to an embodiment of the present disclosure were installed at a specific distance from the containers, and then lights were sequentially irradiated. Thereafter, the bacterial solutions irradiated with the lights were diluted, applied evenly bacterial culture media, and incubated for one day at 35 to 37 °C. Colonies formed on the bacterial culture media were checked, multiplied by a dilution factor, and counted. Therefore, the results for sterilization effects were obtained.

An x-axis shows doses of the first and second lights and a y-axis shows degree of inactivation of bacteria on a logarithmic scale. In Comparative Example 1, only the second light of 275 nm wavelength band was applied to the bacteria. In Comparative Example 2, only the first light of 405 nm wavelength band was applied to the bacteria. In Comparative Example 3, the first light of the 405 nm wavelength band was applied to the bacteria after the second light of the 275 nm wavelength band. In Example, the second light of the 275 nm wavelength band was applied to the bacteria after the first light of the 405 nm wavelength band was applied. Meanwhile, in the graph, in Comparative Example 1, the second light of the wavelength band of 275 nm was applied only with a dose of 3 mJ/cm², and in Comparative Example 2, Comparative Example 3, and Example, respectively, the first light of the wavelength band of 405 nm was applied and changed at doses of 30 mJ/cm², 60 mJ/cm², 90 mJ/cm², 120 mJ/cm², and 150 mJ/cm², respectively and the second light of the wavelength band of 275 nm was applied with a dose of 30 mJ/cm². Here, in the case of the second light, the dose is set lower than that of the first light in consideration of the allowable dose to the human body.

Referring to Figure 10, when only the second light was applied to the bacteria at a dose of 3 mJ/cm² in Comparative Example 1, the degree of inactivation was about 1.5 log CFU/ml. When only the first light was applied to the bacteria at a dose of 30 mJ/cm² in Comparative Example 2, the degree of inactivation was about 1 log CFU/ml. When the second light was pre-irradiated with the bacteria at a dose of 3 mJ/cm² and the first light was post-irradiated with a dose of 30 mJ/cm², the degree of inactivation was about 1.5 log CFU/ml. However, in the embodiment in which the first light was irradiated with a dose of 30 mJ/cm² and the second light was irradiated with a dose of 3 mJ/cm², the deactivation degree was about 4 log CFU/ml and the sterilization effect was very high. Here, in Comparative Example 3 and Example, the order of the first light and the second light were different but the same dose was irradiated to the bacteria. It was seen that the actual sterilization degree showed a significant difference in effect.

In addition, in Comparative Example 2, Comparative Example 3, and Example, the first light of a dose of 60 mJ/cm² were applied. The sterilization effect of Example was significantly higher than Comparative Example 2, or Comparative Example 3.

Meanwhile, when the dose of the first light was more than 90 J/cm², Comparative Example 3 and Example showed a stagnant value of about 6 log CFU/ml. This is because there are no more bacteria to be sterilized under laboratory conditions without introduction of new bacteria. Accordingly, it may be expected that the sterilization effect of Example will be significantly higher than the sterilization effect of Comparative Examples 1 to 3 under open external conditions in which new bacteria are continuously introduced.

Table 1 below shows a minimum dose in order to obtain a desired degree of sterilization in Comparative Examples 1 to 3 and Example. Here, in Comparative Example 1, only the second light of 275 nm wavelength band was applied to the bacteria. In Comparative Example 2, only the first light of 405 nm wavelength band was applied to the bacteria. In Comparative Example 3, the first light of the 405 nm wavelength band was applied to the bacteria after the second light of the 275 nm wavelength band. In Example, the second light of the 275 nm wavelength band was applied to the bacteria after the first light of the 405 nm wavelength band was applied.

Referring to Table 1, in the case of Comparative Example 1 using the second light source alone, a sterilization effect of 90% or more, 99% or more, or 99.9% or more even might be obtained with a very small dose. However, since the influence on the human body is large in the case of the second light, it is difficult to proceed with sterilization by only raising a dose of the second light.

Next, in Comparative Example 2, Comparative Example 3 and Example using a combination of the first light and the second light, it was seen that Example had higher sterilization effect with a very small dose of the first light in comparison with those of Comparative Example 2 and Comparative Example 3. For example, in order to obtain a sterilization effect of 99%, a dose of 65 J/cm² was required for Comparative Example 2 and a dose of 40 J/cm² was required for Comparative Example 3, whereas only a dose of 15 J/cm2 was required for Example.

**[Table 1]**

| Sterilization performance | Example | Comparative Example 1 | Comparative Example 2 | Comparative Example 3 |
|---|---|---|---|---|
| 1 log sterilization (90% sterilization) | 7J/cm² ±10% | 2mJ/cm² ±10% | 30J/cm² ±10% | 20J/cm² ±10% |
| 2 log sterilization (99% sterilization) | 15J/cm² ±10% | 4mJ/cm² ±10% | 65J/cm² ±10% | 40J/cm² ±10% |
| 3 log sterilization (99.9% sterilization) | 25J/cm² ±10% | 8mJ/cm² ±10% | 110J/cm² ±10% | 60J/cm² ±10% |

As described above, it was seen that the light emitting device according to the embodiments of the present disclosure exhibited a significantly higher sterilization effect than the conventional invention.

### Experimental Example 2- Individual Sterilization Power Test of The First Light and The Second Light

In the present test, an MRSA strain was used as a pathogen. After the MRSA strain was cultured, a suspension having a constant bacteria concentration (7 log) was prepared. The first light and the second light were irradiated to the bacteria suspension in each light amount. In this case, the wavelength of the first light was 405 nm and the wavelength of the second light was 275 nm. The bacteria irradiated with the first light and the second light were each diluted at a specific concentration, inoculated into agar plates, and then cultured again. Thereafter, the number of colonies of the cultured bacteria was identified, and the numerical value was converted into a log value. Each test was performed under the same conditions five times.

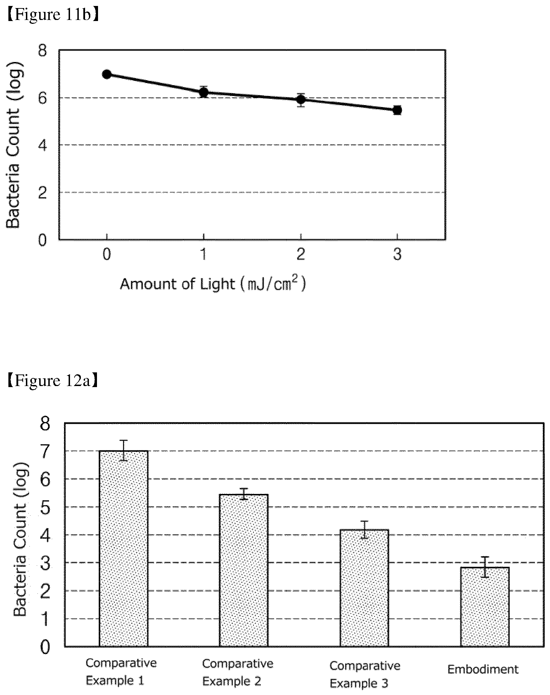

Table 2 and Figure 11a illustrate the test result for the sterilization power of the first light, and Table 3 and Figure 11b illustrate the test result for the sterilization power of the second light.

**[Table 2]**

| Light amount (J/cm²) | 0 | 30 | 60 | 90 | 120 |
|---|---|---|---|---|---|
| Bacteria Count | 7.00 | 5.97 | 5.78 | 5.15 | 4.17 |
| Error | 0.00 | 0.32 | 0.35 | 0.43 | 0.29 |

It may be recognized from Table 2 and Figure 11a that, as an amount of the first light, which is irradiated, is increased, the bacteria count is reduced. It is clear that the bacteria count is reduced even if the margin of error is considered.

**[Table 3]**

| Light amount (J/cm²) | 0 | 1 | 2 | 3 |
|---|---|---|---|---|
| Bacteria Count | 7.00 | 6.23 | 5.88 | 5.45 |
| Error | 0.00 | 0.23 | 0.27 | 0.18 |

It may be recognized from Table 3 and Figure 11b that, as an amount of the second light, which is irradiated, is increased, the bacteria count is reduced. It is clear that the bacteria count is reduced even if the margin of error is considered. In addition, it is recognized that the second light sterilizes the bacteria with an amount smaller than an amount of the first light.

### Experimental Example 3- Sterilization Power Test In The Combination Of The First Light And The Second Light

In the present test, an MRSA strain was used as a pathogen. After the MRSA strain was cultured, a suspension having a constant bacteria concentration (7 log) was prepared. The individual irradiation of the first light, the individual irradiation of the second light, and the combination of the first light and the second light were performed with respect to the bacteria suspension. Comparative Example 1 illustrates that non-light is irradiated to the bacteria suspension, Comparative example 2 illustrates that the second light was individually irradiated to the bacteria suspension, Comparative example 3 illustrates that the first light was individually irradiated to the bacteria suspension, and Embodiment illustrates that the combination of the first light and the second light was irradiated to the bacteria suspension. In this case, the wavelength of the first light was 405 nm, the dose of the first light was 120 J/cm², and the wavelength of the second light was 275 nm, and the dose of the second light was 3 mJ/cm². In Embodiment, the second light was irradiated in the dose of 3 mJ/cm² and then the first light was irradiated in the dose of 120 J/cm². Next, in Comparative Examples 1 to 3 and Embodiment, the bacteria were diluted at a constant concentration, inoculated into agar plates, and then cultured again. Thereafter, the number of colonies of the cultured bacteria was identified, and the numerical value was converted into a log value.

Each test was performed under the same conditions five times.

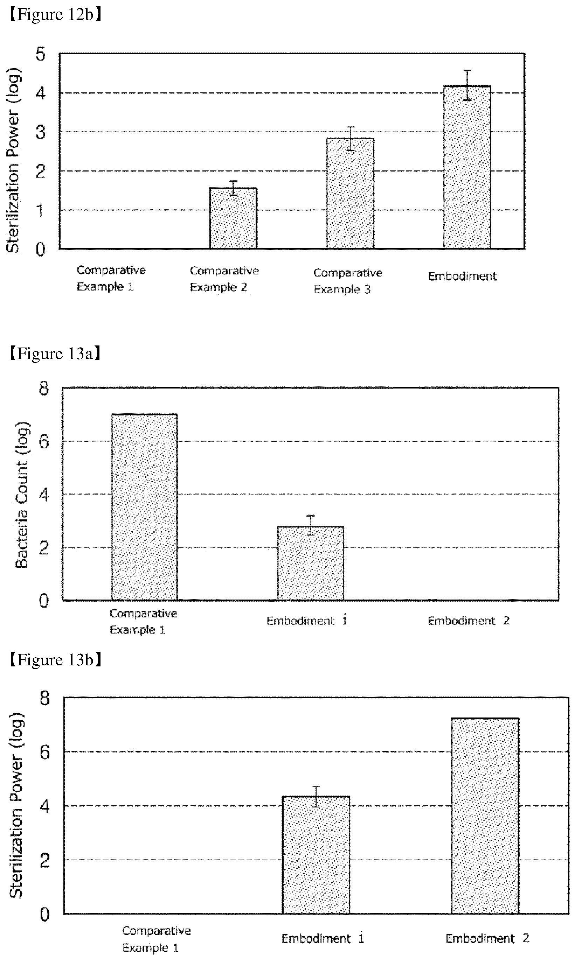

Figure 12a and Table 4 illustrate the bacteria count in the individual irradiation of the first light, the individual irradiation of the second light, and the irradiation of the combination of the first light and the second light. Figure 12b and Table 5 illustrate the sterilization power in the individual irradiation of the first light, the individual irradiation of the second light, and the irradiation of the combination of the first light and the second light.

**[Table 4]**

| Light condition | Comparative Example 1 | Comparative Example 2 | Comparative Example 3 | Embodiment |
|---|---|---|---|---|
| Bacteria Count | 7.00 | 5.45 | 4.17 | 2.83 |
| Error | 0.00 | 0.18 | 0.29 | 0.37 |

**[Table 5]**

| Light condition | Comparative Example 1 | Comparative Example 2 | Comparative Example 3 | Embodiment |
|---|---|---|---|---|
| Sterilization power | 0.00 | 1.55 | 2.83 | 4.17 |
| Error | 0.00 | 0.18 | 0.29 | 0.37 |

Referring to Figures 12a, 12b, Table 4, and Table 5, about 90% of sterilization power was illustrated in the individual irradiation of the second light, about 99% of sterilization power was illustrated in the individual irradiation of the first light, and 99.99% or more of sterilization power was illustrated in irradiation of the combination of the first light and the second light. Accordingly, it may be recognized that an amount of bacteria is significantly reduced, and thus the sterilization power is significantly increased when the combination of the first light and the second light is irradiated, as compared to when the light is not irradiated, and to when the first light or the second light is individually irradiated.

### Experimental Example 4-Test For Variation in Sterilization Power Based On Sequence of Combining The First Light and The Second Light

In the present test, an MRSA strain was used as a pathogen. After the MRSA strain was cultured, a suspension having a constant bacteria concentration (7 log) was prepared. After the second light was irradiated to the bacteria suspension, the first light was irradiated to the bacteria suspension. In addition, the second light was irradiated to the bacteria suspension after the first light was irradiated to the bacterial suspension. Comparative example 1 illustrates that non-light was irradiated to the bacteria suspension, Embodiment 1 illustrates that the first light was irradiated to the bacteria suspension after the second light was irradiated to the bacteria suspension, and Embodiment 2 illustrates that the second light was irradiated to the bacteria suspension after the first light was irradiated to the bacteria suspension.

In Embodiment 1, after the second light having the wavelength of 275 nm was irradiated to the bacteria suspension with a dose of 3 mJ/cm², the first light having the wavelength of 405 nm was irradiated to the bacteria suspension with a dose of 120 J/cm². In Embodiment 2, after the first light having the wavelength of 405 nm was irradiated to the bacteria suspension with a dose of 120 J/cm², the second light having the wavelength of 275 nm was irradiated with the dose of 3 mJ/cm².

Next, in Comparative Example, Embodiment 1, and Embodiment 2, the bacteria were diluted at a constant concentration, inoculated into agar plates, and then cultured again. Thereafter, the number of colonies of the cultured bacteria was identified, and the numerical value was converted into a log value.

Each test was performed under the same conditions five times.

Figure 13a and Table 6 illustrate the bacteria count when the sequence of combining the first light and the second light is differently set, and Figure 13b and Table 7 illustrate the sterilization power when the sequence of combining the first light and the second light is differently set.

**[Table 6]**

| Light condition | Comparative Example | Embodiment 1 | Embodiment 2 |
|---|---|---|---|
| Bacteria Count | 7.00 | 2.83 | 0.00 |
| Error | 0.00 | 0.37 | 0.00 |

**[Table 7]**

| Light condition | Comparative Example | Embodiment 1 | Embodiment 2 |
|---|---|---|---|
| Sterilization power | 0.00 | 4.17 | 7.00 |
| Error | 0.00 | 0.37 | 0.00 |

It may be recognized from Figures 13a and 13b, Table 6, and Table 7 that Embodiment 1 illustrates 99.99% of sterilization power, and bacteria are not observed in Embodiment 2, so the sterilization is substantially completely achieved. In other words, the case that the second light is irradiated after the first light is irradiated shows significantly higher sterilization power with the same irradiation amount of light, as compared to the case the first light is irradiated after the second light is irradiated, which means that the same sterilization power is obtained with a smaller amount of light as compared to the case the first light is irradiated after the second light is irradiated. The application of a smaller amount of light means the reduction in the light irradiation time. Accordingly, Embodiment 2 is more reduced in the light irradiation time than Embodiment 1.

### Experimental Example 5 - Setting Condition of An Amount of Light (In Vitro)

The bacteria count and the sterilization power were measured as function of an amount of light in vitro condition when the first light and the second light are sequentially irradiated, in order to find out the optimal amount of each light, based on that the sequential irradiation of the first light and the second light, which shows the increase in the sterilization power.

In the present test, an MRSA strain was used as a pathogen. After the MRSA strain was cultured, a suspension having a constant bacteria concentration (7 log) was prepared. The first light and the second light were sequentially irradiated to the bacteria suspension by changing the dose of the first light to 30 J/cm², 60 J/cm², 90 J/cm², and 120 J/cm². However, in the case of the second light, the light of 275 nm was employed with a dose limited to 3 mJ/cm² based on the allowable level of the human body.

Next, the bacteria were diluted at a constant concentration, inoculated into agar plates, and then cultured again. Thereafter, the number of colonies of the cultured bacteria was identified, and the numerical value was converted into a log value.

Each test was performed under the same conditions five times.

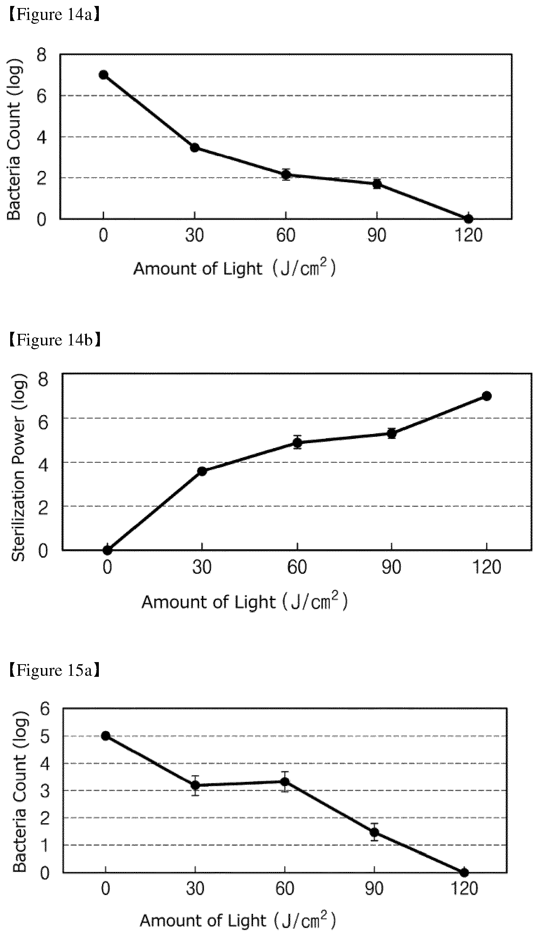

Figure 14a and Table 8 show the bacteria count when an amount of the first light is variably set while the first light and the second light are sequentially irradiated, and Figure 14b and Table 9 show the sterilization power when an amount of the first light is variably set while the first light and the second light are sequentially irradiated.

**[Table 8]**

| Light amount (J/cm²) | 0 | 30 | 60 | 90 | 120 |
|---|---|---|---|---|---|
| The number of bacteria | 7.00 | 3.47 | 2.13 | 1.70 | 0.00 |
| Error | 0.00 | 0.13 | 0.27 | 0.22 | 0.00 |

**[Table 9]**

| Light amount (J/cm²) | 0 | 30 | 60 | 90 | 120 |
|---|---|---|---|---|---|
| Sterilization power | 0.00 | 3.53 | 4.87 | 5.03 | 7.00 |
| Error | 0.00 | 0.13 | 0.27 | 0.22 | 0.00 |

It may be recognized from Figures 14a, 14b, Table 8, and Table 9 that the number of bacteria is reduced as an amount of the first light is increased and the sterilized is completely achieved with a dose of 120 J/cm².

### Experimental Example 6 - Setting Of Light Amount Condition (In Vivo)

It was recognized through Embodiment 4 that the sterilization is completely achieved when a dose of the first light (having the wavelength of 275 nm) is 120 J/cm², under the condition that the dose of the second light (having the wavelength of 405 nm) is 3 mJ/cm². Accordingly, the test was performed to determine whether the above sterilization effect is obtained under in vivo condition.

The present test was performed using a mouse to determine whether the application of light is effective and safe under in vivo condition. The condition for an amount of light is set to the same condition as that in vitro. For a mouse, a BALB/c mouse (6-8 weeks old) was used, the back of the mouse was shaved, and then a cut was formed in the diameter of 10 mm in the back of the mouse. After the pathogenic bacteria was inoculated (at 5 log) on the wound, the first light and the second light were sequentially irradiated by changing the dose of the first light to 30 J/cm², 60 J/cm2, 90 J/cm2, and 120 J/cm2. However, in the case of the second light, the light of 275 nm was employed with a dose limited to 3 mJ/cm² based on the allowable level of the human body. Next, tissues were sampled, and the sampled tissues were disrupted, diluted at a predetermined concentration, inoculated on agar plates, and then cultured again. Thereafter, the number of colonies of the cultured bacteria was identified, and the numerical value was converted into a log value.

Each test was performed under the same conditions five times.

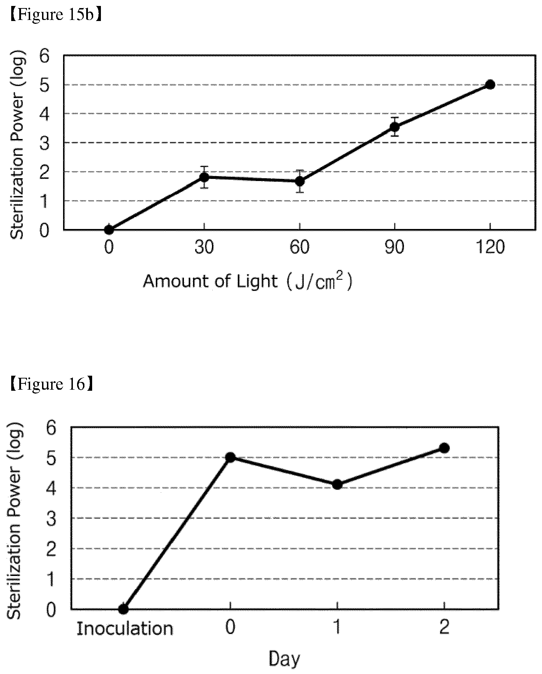

Figure 15a and Table 10 show the bacteria count as a function of an amount of the first light, when the first light and the second light were sequentially irradiated. Figure 15b and Table 11 show the sterilization power as a function of an amount of the first light, when the first light and the second light were sequentially irradiated.

**[Table 10]**

| Light amount (J/cm²) | 0 | 30 | 60 | 90 | 120 |
|---|---|---|---|---|---|
| Bacteria Count | 5.00 | 3.17 | 3.32 | 1.48 | 0.00 |
| Error | 0.00 | 0.36 | 0.38 | 0.31 | 0.00 |

**[Table 11]**

| Light amount (J/cm²) | 0 | 30 | 60 | 90 | 120 |
|---|---|---|---|---|---|
| Sterilization power | 0.00 | 1.83 | 1.68 | 3.52 | 5.00 |
| Error | 0.00 | 0.36 | 0.38 | 0.31 | 0.00 |

It may be recognized from Figures 15a, 15b, Table 10, and Table 11 that the bacteria count is reduced as an amount of the first light is increased under in vivo condition and the sterilized is completely achieved with a dose of 120 J/cm².

### Experimental Example 7 - Effectiveness Evaluation 1 (in vivo)

In embodiment 5, a dose of light for sterilization was recognized under in vivo condition, and the variation in the sterilization power and the variation in the bacteria count as functions of time were tested under in vivo condition.

The present test was performed using a mouse. For the mouse, a BALB/c mouse (6-8 weeks old) was used, the back of the mouse was shaved, and then a cut was formed in the diameter of 10 mm in the back of the mouse. After the pathogenic bacteria was inoculated (at 5 log) on the wound, the first light and the second light were sequentially irradiated and repeatedly irradiated six times in total at the same time every day while a dose of the first light (having the wavelength of 405 nm) is 120 J/cm². In the case of the second light, the light of 275 nm was employed with a dose limited to 3 mJ/cm² based on the allowable level of the human body.

Next, to determine the bacteria count every day, tissues were sampled, and the sampled tissues were disrupted, diluted at a predetermined concentration, inoculated on agar plates, and then cultured again. Thereafter, the number of colonies of the cultured bacteria was identified, and the numerical value was converted into a log value. To determine the initial sterilization power, the bacteria count was detected until three-time light irradiation.

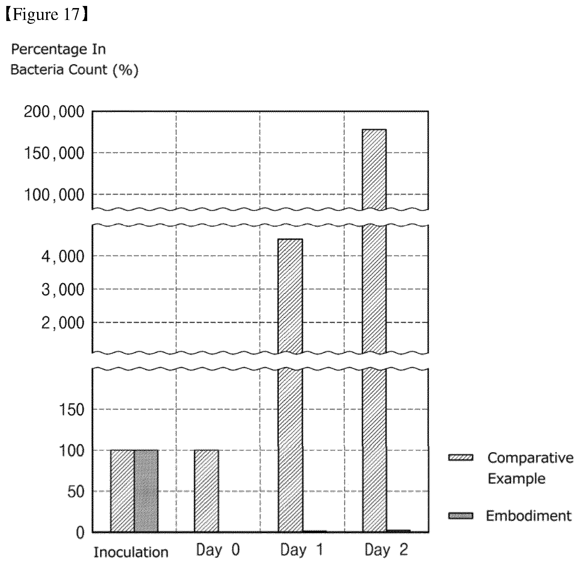

Figure 16 and Table 12 show the variation in the sterilization power depending on days under in vivo condition, and Figure 17 and Table 13 show the measurement result of the number of bacteria in each day under in vivo condition. In Figure 17 and Table 13, Comparative example is a non-irradiation group without light irradiation, and Embodiment corresponds to a light irradiation group irradiated with light.

**[Table 12]**

| Day | Inoculation | 0 | 1 | 2 |
|---|---|---|---|---|
| Sterilization power | 0.00 | 5.00 | 4.09 | 5.29 |
| Error | 0.00 | 0.00 | 0.13 | 0.09 |

**[Table 13]**

| | The number of bacteria (%) | | | | The number of bacteria (log) | | | |
|---|---|---|---|---|---|---|---|---|
| Day | Inoculation | 0 | 1 | 2 | Inoculation | 0 | 1 | 2 |
| Non-irradiation group | 100 | 100 | 4,466 | 173,780 | 5.00 | 5.00 | 6.65 | 8.24 |
| Light irradiation group | 100 | 0 | 0.36 | 0.89 | 5.00 | 0.00 | 2.56 | 2.95 |

It may be recognized from Figure 16, Figure 17, Table 12, and Table 13 that the sterilization power is continuously maintained to 99.99% or more after light is irradiated to the wound at the initial stage, and the number of bacteria is substantially approximate to '0' when the light is irradiated.

### Experimental Example 8 - Effectiveness Evaluation 2 (in vivo)

In Embodiment 5, a dose of light for sterilization was recognized under in vivo condition, and the effect of treating the wound by irradiating the light was tested under in vivo condition based on the dose of light for sterilization.

The present test was performed using a mouse. For the mouse, a BALB/c mouse (6-8 weeks old) was used, the back of the mouse was shaved, and then a wound was formed in the diameter of 10 mm in the back of the mouse. After the pathogenic bacteria was inoculated (at 5 log) on the wound, the first light and the second light were sequentially irradiated and repeatedly irradiated six times in total at the same time every day while a dose of the first light (having the wavelength of 405 nm) is 120 J/cm². However, in the case of the second light, the light of 275 nm was employed with a dose limited to 3 mJ/cm² based on the allowable level of the human body.

The variation in the shape (especially, an area) of the wound was observed at the same time every day. The size of the wound was observed every day till epithelialization, and the value thereof was recorded.

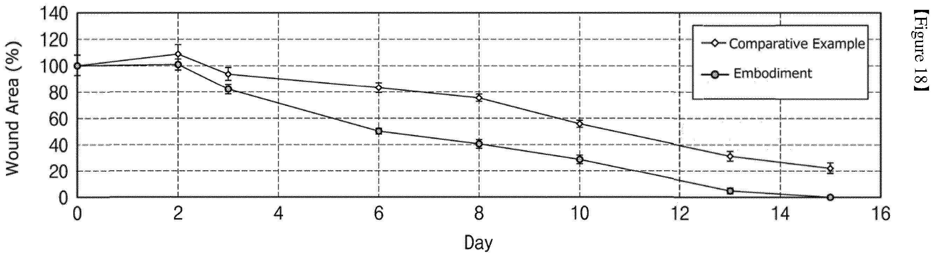

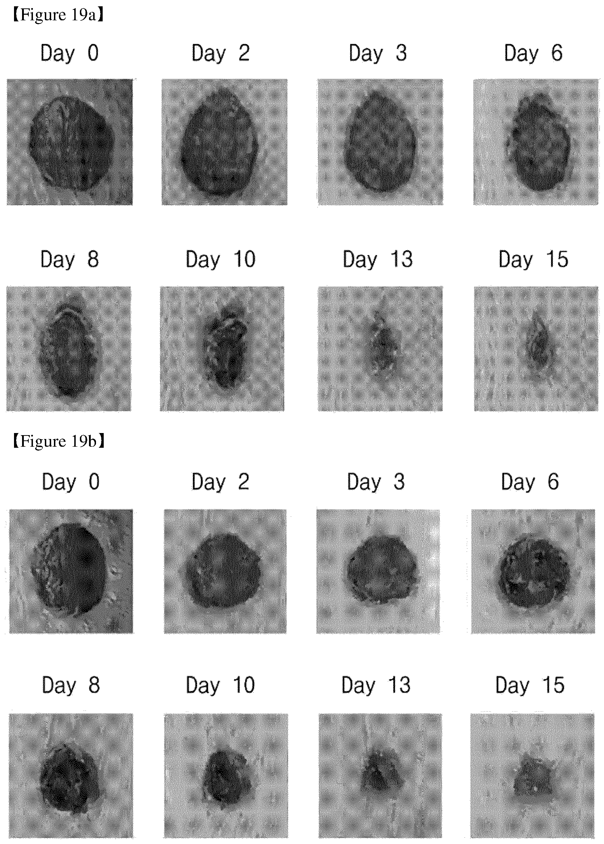

Figure 18 and Table 14 show the variation in the area of a wound depending on days under in vivo condition. In Figure 18 and Table 14, Comparative example is a non-irradiation group without light irradiation, and Embodiment corresponds to a light irradiation group irradiated with light. Figures 19a and 19b are photographs obtained by capturing images of the shape of the wound area depending on days. Figure 19a illustrates photographs of a wound in the non-irradiation group, and Figure 19b illustrates photographs of wounds in the light irradiation group.

**[Table 14]**

| Day | Inoculation | 0 | 2 | 3 | 6 | 10 | 15 |
|---|---|---|---|---|---|---|---|
| Non-irradiation group | 100.0 | 100.0 | 108.8 | 93.8 | 83.3 | 55.9 | 22.4 |
| Error | 7.8 | 7.8 | 7.0 | 5.0 | 3.8 | 2.7 | 4.2 |
| Light irradiation group | 100.0 | 100.0 | 101.0 | 82.1 | 50.3 | 28.8 | 0.0 |
| Error | 7.8 | 7.8 | 4.1 | 3.6 | 1.9 | 3.2 | 0.0 |

Referring to Figure 18, Table 14, Figure 19a, and Figure 19b, the wound cured was not visibly observed until 2 days from the wound, and the bacteria count in the wound was significantly reduced. Accordingly, it was determined that the sterilization was in progress. A scab was produced from 2 days after the wound and then the area of the wound was gradually reduced. Accordingly, the curing of the wound is in progress from 2 days after the wound. When the scab was produced on the wound, the wound exposed to the outside was disappeared by the scab. Therefore, the additional infection is less caused. However, the size of the scab and the recovery rate of the wound greatly varied depending on the sterilization state until the scab was formed. Although the light irradiation group required 6 days till a time point at which the area of the wound was reduced to 50% in the stage of curing the wound, the non-irradiation group required 10 days till the time point. Further, the epithelialization was achieved on the 15^{th} day in the case of the light irradiation group, and not achieved in the case of the non-irradiation group. Accordingly, according to an embodiment of the present disclosure, it may be recognized that the effect of curing the wound is significantly produced when light is irradiated.

### Experimental Example 9 - Safety Evaluation 1 (in vivo)

In the above-described experimental example, a DNA mutation state was determined to determine whether the irradiation condition is harmful to the human body.

In the present test, to determine whether the DNA mutation was caused to the tissue which is not infected through light irradiation, the formation degree of a thymine dimer was determined through immunohistochemical analysis. When an excessive amount of UV is irradiated to the DNA, the DNA mutation such as the thymine dimer is caused, so the cell is destroyed. Accordingly, the DNA mutation may be determined based on the formation degree of the thymine dimer.

The present test was performed using a mouse. For the mouse, a BALB/c mouse (6-8 weeks old) was used, the back of the mouse was shaved, and then a wound is formed in the diameter of 10 mm in the back of the mouse by using the punch. After light was irradiated on the wound, the tissue was sampled, the sampled tissue was fixed through formalin and paraffin, and a cut-out fragment was taken. When light was irradiated, the control group was a non-irradiation group in which light was not treated, Experimental group 1 was a light irradiation group in which an excessive amount of UVC was treated, Experimental group 2 was a light irradiation group in which the first light and the second light were sequentially irradiated in the state that a dose of the first light (having the wavelength of 405 nm) is limited to 120 J/cm² and, a dose of the second light (having the wavelength of 275 nm) is limited to 3 mJ/cm².

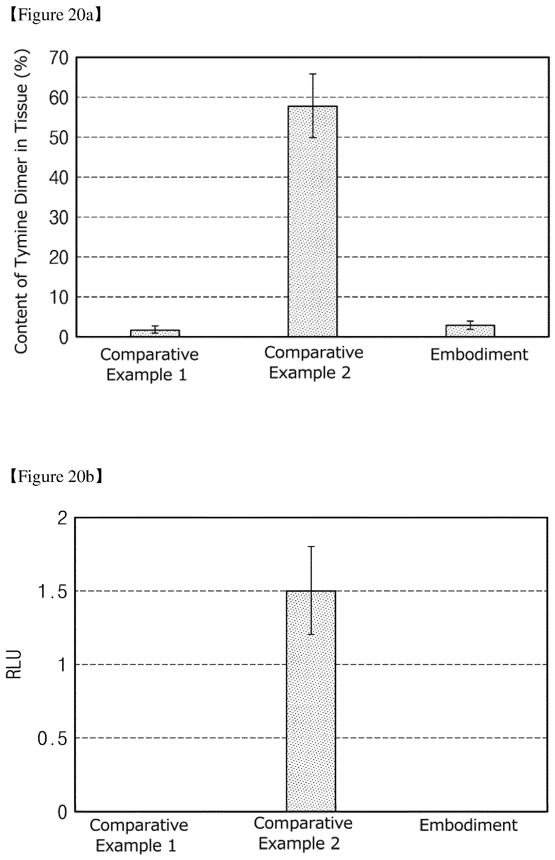

Figure 16a and Table 15 illustrate the percentage of a thymine dimer in a tissue. Referring to Figure 20a and Table 15, although the thymine dimer was found in Experimental group 1, the thymine dimer was not found in Experimental group 2. Accordingly, under the light condition applied according to the embodiments of the present disclosure, it was determined that the DNA mutation was not found even if the light was irradiated to a tissue which was not infected.

**[Table 15]**

| | Control group | Experimental group 1 | Experimental group 1 |
|---|---|---|---|
| Content (%) | 2 | 58 | 3 |
| Error | 1 | 8 | 1 |

### Experimental Example 10 - Safety Evaluation 1 (in vivo)

In the above-described experimental example, the generation state of ROS was determined to determine whether the irradiation condition was harmful to the human body.

The present test is to determine whether the ROS was induced even in the tissue, which was not infected, through light irradiation. When the sterilizing light was irradiated to the infectious bacteria, the ROS was induced to destroy the bacteria.

The present test was performed using a mouse. For the mouse, a BALB/c mouse (6-8 weeks old) was used, the back of the mouse was shaved, and then a wound is formed in the diameter of 10 mm in the back of the mouse by using the punch. After the light was irradiated on the wound, a Dichlorofluorescin diacetate (DCFH-DA) was treated for a part irradiated with light and a light emission was measured with respect to a part stained with DCFH-DA, so it was determined that the ROS was present. DCFH-DA was oxidized by the ROS in the cell to emit fluorescent light. When DCFH-DA was excited, the absorption wavelength was in the range of 445 nm to 490 nm, and the fluorescent wavelength was in the range of 515 nm to 575 nm.

In this case, the control group was a non-treatment group in which non-treatment is added, Experimental group 1 was a group treated with hydrogen peroxide, and Experimental group 2 was a treatment group to which the first light and the second light are sequentially irradiated in the state that a dose of the first light (having the wavelength of 405 nm) is limited to 120 J/cm², and a dose of the second light (having the wavelength of 275 nm) is limited to 3 mJ/cm².

Figure 20b and Table 16 illustrate the light emission degree of a part stained with DCFH-DA. Referring to Figure 20b and Table 16, fluorescent is emitted in Experimental group 2 and Experimental group 1, so it is determined that ROS is present. However, since fluorescent is not absent in Experiment group 2, so it is determined that ROS is absent. Accordingly, under the light condition applied according to the embodiments of the present disclosure, it was determined that the ROS was not produced even if the light was irradiated to a tissue which was not infected.

**[Table 16]**

| | Control group | Experimental group 1 | Experimental group 2 |
|---|---|---|---|
| Light emission degree (RLU; relative light units) | 0 | 1.5 | 0 |
| Error | 0 | 0.3 | 0 |

The invention is defined in the following claims.

## Claims

1. A light irradiation device (100) comprising:
a housing;
a substrate (20) provided in the housing; and
a light source mounted on the substrate,
wherein the light source includes:
a first light source (30) including at least one first light source and configured to emit first light (L1) having a blue wavelength band;
a second light source (40) including at least one second light source and configured to emit second light (L2) having a ultraviolet wavelength band; and
a control unit (50) that controls light emission of the first light source and the second light source such that the first light and the second light are emitted sequentially, regardless of whether emission of the first light overlaps with emission of the second light, or not, wherein when the first light and the second light are sequentially irradiated, the first light (L1) is first irradiated, and then the second light (L2) is irradiated, such that one of a) irradiation of the second light (L2) starts after the irradiation of the first light (L1) is finished or b) the irradiation of the second light (L2) starts even though the irradiation of the first light (L1) is not finished, wherein the control unit controls the first light source and the second light source to emit the second light after starting to emit the first light,
wherein a dose of the second light source is less than 1/10 of a dose of the first light source,
the light irradiation device further including an optical unit to selectively collect or radiate light emitted from the first and second light sources.

2. The light irradiation device of claim 1, wherein the second light corresponds to at least one of UVA (ultraviolet A), UVB (ultraviolet B), and UVC (Ultraviolet C) wavelength bands.

3. The light irradiation device of claim 1, wherein the first light has a wavelength band in a range of about 400 nm to about 500 nm.

4. The light irradiation device of claim 1, wherein the first light further includes light having a wavelength band corresponding to a visible light.

5. The light irradiation device of claim 1, wherein the second light has a wavelength band in a range of about 240 nm to about 280 nm.

6. The light irradiation device of claim 5, wherein the first light is irradiated for a first time, and the second light is irradiated for a second time shorter than the first time.

7. The light irradiation device of claim 6, wherein the second light starts to be irradiated after the first light stops irradiation.

8. The light irradiation device of claim 6, wherein the second light starts to be irradiated before the first light stops irradiation, and at least a portion of the first time and the second time has a mutually overlapping duration.

9. The light irradiation device of claim 6, wherein the first light is continuously irradiated.

10. The light irradiation device of claim 6, wherein the second light is discontinuously irradiated.

11. The light irradiation device of claim 6, wherein the second light is periodically irradiated.

12. The light irradiation device of claim 1, wherein the light irradiation device is used to treat acute wound.

13. The light irradiation device of claim 1, wherein the second light source emits the second light within a predetermined dose per day, and the predetermined dose per day is in a harmless range to be an allowable dose when the second light is applied to a human body.

14. The light irradiation device of claim 13, wherein the second light is irradiated with a dose in a range of about 30 J/m² to about 1,000,000 J/m².

## Patentansprüche

1. Lichtbestrahlungsvorrichtung (100), die folgende Komponenten umfasst:
ein Gehäuse;
ein im Gehäuse vorgesehenes Substrat (20); und
eine auf dem Substrat montierte Lichtquelle,
wobei die Lichtquelle umfasst:
eine erste Lichtquelle (30), die mindestens eine erste Lichtquelle umfasst und so ausgelegt ist, dass sie erstes Licht (L1) mit einem blauen Wellenlängenbereich emittiert;
eine zweite Lichtquelle (40), die mindestens eine zweite Lichtquelle umfasst und so ausgelegt ist, dass sie zweites Licht (L2) mit einem ultravioletten Wellenlängenbereich emittiert; und
eine Steuereinheit (50), die die Lichtemission der ersten Lichtquelle und der zweiten Lichtquelle so steuert, dass das erste Licht und das zweite Licht nacheinander emittiert werden, unabhängig davon, ob sich die Emission des ersten Lichts mit der Emission des zweiten Lichts überschneidet oder nicht, wobei , wenn das erste Licht und das zweite Licht nacheinander ausgestrahlt werden, zunächst das erste Licht (L1) und anschließend das zweite Licht (L2) ausgestrahlt wird, sodass entweder a) die Ausstrahlung des zweiten Lichts (L2) beginnt, nachdem die Ausstrahlung des ersten Lichts (L1) beendet ist, oder b) die Bestrahlung mit dem zweiten Licht (L2) beginnt, obwohl die Bestrahlung mit dem ersten Licht (L1) noch nicht beendet ist , wobei die Steuereinheit die erste Lichtquelle und die zweite Lichtquelle so steuert, dass das zweite Licht nach Beginn der Emission des ersten Lichts emittiert wird,
wobei die Dosis der zweiten Lichtquelle weniger als 1/10 der Dosis der ersten Lichtquelle beträgt,
wobei die Lichtbestrahlungsvorrichtung ferner eine optische Einheit umfasst, um von der ersten und der zweiten Lichtquelle emittiertes Licht selektiv zu sammeln oder auszustrahlen.

2. Lichtbestrahlungsvorrichtung nach Anspruch 1, wobei das zweite Licht mindestens einem der Wellenlängenbereiche UVA (Ultraviolett A), UVB (Ultraviolett B) und UVC (Ultraviolett C) entspricht.

3. Lichtbestrahlungsvorrichtung nach Anspruch 1, wobei das erste Licht ein Wellenlängenband im Bereich von etwa 400 nm bis etwa 500 nm aufweist.

4. Lichtbestrahlungsvorrichtung nach Anspruch 1, wobei das erste Licht ferner Licht umfasst, das ein Wellenlängenband aufweist, das dem sichtbaren Licht entspricht.

5. Lichtbestrahlungsvorrichtung nach Anspruch 1, wobei das zweite Licht ein Wellenlängenband im Bereich von etwa 240 nm bis etwa 280 nm aufweist.

6. Lichtbestrahlungsvorrichtung nach Anspruch 5, wobei das erste Licht ein erstes Mal bestrahlt wird und das zweite Licht ein zweites Mal bestrahlt wird, wobei die Dauer des zweiten Mal kürzer ist als die des ersten Mal.

7. Lichtbestrahlungsvorrichtung nach Anspruch 6, wobei die Bestrahlung mit dem zweiten Licht beginnt, nachdem die Bestrahlung mit dem ersten Licht beendet wurde.

8. Lichtbestrahlungsvorrichtung nach Anspruch 6, wobei die Bestrahlung mit dem zweiten Licht beginnt, bevor die Bestrahlung mit dem ersten Licht beendet ist, und sich zumindest ein Teil der ersten und der zweiten Bestrahlungsdauer gegenseitig überlappt.

9. Lichtbestrahlungsvorrichtung nach Anspruch 6, wobei das erste Licht kontinuierlich bestrahlt wird.

10. Lichtbestrahlungsvorrichtung nach Anspruch 6, wobei das zweite Licht diskontinuierlich ausgestrahlt wird.

11. Lichtbestrahlungsvorrichtung nach Anspruch 6, wobei das zweite Licht periodisch ausgestrahlt wird.

12. Lichtbestrahlungsvorrichtung nach Anspruch 1, wobei die Lichtbestrahlungsvorrichtung zur Behandlung akuter Wunden verwendet wird.

13. Lichtbestrahlungsvorrichtung nach Anspruch 1, wobei die zweite Lichtquelle das zweite Licht innerhalb einer vorgegebenen Tagesdosis emittiert und die vorgegebene Tagesdosis in einem unbedenklichen Bereich liegt, sodass sie eine zulässige Dosis darstellt, wenn das zweite Licht auf einen menschlichen Körper einwirkt.

14. Lichtbestrahlungsvorrichtung nach Anspruch 13, wobei das zweite Licht mit einer Dosis in einem Bereich von etwa 30 J/m² bis etwa 1.000.000 J/m² bestrahlt wird.

## Revendications

1. Dispositif d'irradiation lumineuse (100) comprenant :
un boîtier ;
un substrat (20) disposé dans le boîtier ; et
une source lumineuse montée sur le substrat,
dans lequel la source lumineuse comprend :
une première source lumineuse (30) comprenant au moins une première source lumineuse et configurée pour émettre une première lumière (L1) présentant une bande de longueurs d'onde bleue ;
une deuxième source lumineuse (40) comprenant au moins une deuxième source lumineuse et configurée pour émettre une deuxième lumière (L2) dans la bande de longueurs d'onde ultraviolette ; et
une unité de contrôle (50) qui contrôle l'émission lumineuse de la première source lumineuse et de la deuxième source lumineuse de telle sorte que la première lumière et la deuxième lumière soient émises séquentiellement, que l'émission de la première lumière chevauche ou non celle de la seconde lumière, dans lequel lorsque la première lumière et la seconde lumière sont irradiées séquentiellement, la première lumière (L1) est d'abord irradiée, puis la seconde lumière (L2) est irradiée, de telle sorte que soit a) l'irradiation de la seconde lumière (L2) commence après la fin de l'irradiation de la première lumière (L1), soit b) l'irradiation de la deuxième lumière (L2) commence même si l'irradiation de la première lumière (L1) n'est pas terminée , dans lequel l'unité de contrôle commande la première source lumineuse et la deuxième source lumineuse pour qu'elles émettent la deuxième lumière après avoir commencé à émettre la première lumière,
dans lequel la dose de la deuxième source lumineuse est inférieure à 1/10 de la dose de la première source lumineuse,
le dispositif d'irradiation lumineuse comprenant en outre une unité optique destinée à collecter ou à rayonner de manière sélective la lumière émise par les première et deuxième sources lumineuses.

2. Dispositif d'irradiation lumineuse selon la revendication 1, dans lequel la seconde lumière correspond à au moins l'une des bandes de longueurs d'onde UVA (ultraviolet A), UVB (ultraviolet B) et UVC (ultraviolet C).

3. Dispositif d'irradiation lumineuse selon la revendication 1, dans lequel la première lumière présente une plage de longueurs d'onde comprise entre environ 400 nm et environ 500 nm.

4. Dispositif d'irradiation lumineuse selon la revendication 1, dans lequel la première lumière comprend en outre une lumière ayant une bande de longueurs d'onde correspondant à la lumière visible.

5. Dispositif d'irradiation lumineuse selon la revendication 1, dans lequel la deuxième lumière présente une plage de longueurs d'onde comprise entre environ 240 nm et environ 280 nm.

6. Dispositif d'irradiation lumineuse selon la revendication 5, dans lequel la première lumière est émise une première fois, et la deuxième lumière est émise une deuxième fois pendant une durée plus courte que la première fois.

7. Dispositif d'irradiation lumineuse selon la revendication 6, dans lequel la seconde lumière commence à être émise après que la première lumière a cessé d'être émise.

8. Dispositif d'irradiation lumineuse selon la revendication 6, dans lequel la deuxième lumière commence à être émise avant que la première lumière ne cesse d'être émise, et au moins une partie de la première durée et de la deuxième durée se chevauchent.

9. Dispositif d'irradiation lumineuse selon la revendication 6, dans lequel la première lumière est émise en continu.

10. Dispositif d'irradiation lumineuse selon la revendication 6, dans lequel la deuxième lumière est émise de manière discontinue.

11. Dispositif d'irradiation lumineuse selon la revendication 6, dans lequel la deuxième lumière est émise de manière périodique.

12. Dispositif d'irradiation lumineuse selon la revendication 1, dans lequel le dispositif d'irradiation lumineuse est utilisé pour traiter une plaie aiguë.

13. Dispositif d'irradiation lumineuse selon la revendication 1, dans lequel la deuxième source lumineuse émet la deuxième lumière dans les limites d'une dose journalière prédéterminée, et ladite dose journalière prédéterminée se situe dans une plage inoffensive de sorte à constituer une dose admissible lorsque la deuxième lumière est appliquée sur un corps humain.

14. Dispositif d'irradiation lumineuse selon la revendication 13, dans lequel la deuxième lumière est irradiée à une dose comprise dans une plage allant d'environ 30 J/m² à environ 1 000 000 J/m².
